(19) Europäisches Patentamt
European Patent Office
Office européen des brevets

(11) Veröffentlichungsnummer: **0 273 190 B1**

# (12) EUROPÄISCHE PATENTSCHRIFT

(45) Veröffentlichungstag der Patentschrift: **17.04.91**

(51) Int. Cl.5: **C07F 9/547**, A61K 31/675

(21) Anmeldenummer: **87117191.4**

(22) Anmeldetag: **21.11.87**

Die Akte enthält technische Angaben, die nach dem Eingang der Anmeldung eingereicht wurden und die nicht in dieser Patentschrift enthalten sind.

## (54) Neue Diphosphonsäurederivate, Verfahren zu deren Herstellung und diese Verbindung enthaltende Arzneimittel.

(30) Priorität: **29.11.86 DE 3640938**

(43) Veröffentlichungstag der Anmeldung:
**06.07.88 Patentblatt 88/27**

(45) Bekanntmachung des Hinweises auf die Patenterteilung:
**17.04.91 Patentblatt 91/16**

(84) Benannte Vertragsstaaten:
**AT BE CH DE ES FR GB GR IT LI LU NL SE**

(56) Entgegenhaltungen:
**EP-A- 258 168**
**EP-A- 274 158**
**EP-A- 0 001 584**
**EP-A- 0 186 405**
**EP-A- 272 208**
**EP-A- 275 821**
**EP-A- 0 170 228**

**CHEMICAL ABSTRACTS, Band 99, Nr. 3, 18. Juli 1983, Seite 640, Spalte 2, Zusammenfassungsnr. 22702b, Columbus, Ohio, US; M.I. KABACHNIK et al.: "Gamma-amino-substituted alpha-hydroxypropylidenedi-phosphonic acids", & SU 1 002 300 (INSTITUTE OF HETEROORGANIC COMPOUNDS, ACADEMY OF SCIENCES) 07.03.1983**

**CHEMICAL ABSTRACTS, Band 96, Nr. 8, 8.-22. März 1982, Seite 591, Zusammenfassungsnr. 77483z, Columbus, Ohio, US; & JP - A - 81 97347 (JPN. KOKAI TOKKYO KOHO) 06.08.1981**

(73) Patentinhaber: **BOEHRINGER MANNHEIM GMBH**
**Sandhofer Strasse 116**
**W-6800 Mannheim 31(DE)**

(72) Erfinder: **Bosies, Elmar, Dr. rer. nat.**
**Delpstrasse 11**
**W-6940 Weinheim(DE)**
Erfinder: **Gall, Rudi, Dr. phil.**
**Ulmenstrasse 1**
**W-6945 Hirschberg 1(DE)**

**EP 0 273 190 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Diphosphonsaeurederivate, Verfahren zu deren Herstellung sowie Arzneimittel, die diese Substanzen enthalten.

In der DE-PS 18 13 659 sind Diphosphonsaeurederivate beschrieben, von denen die 1-Hydroxy-ethan-1,1-diphosphonsaeure als Mittel zur Behandlung von Morbus Paget Bedeutung erlangt hat. In der DE-OS 27 45 083 ist u.a. das 1-Hydroxy-1-(pyrrolidin-2-yl)methan-1,1-diphosphonat als Komplexbildner beschrieben. In der Jpn. Kokai Tokkyo Koho 8098.193 sind u.a. Pyridylmethandiphosphonate als Herbizide und in der DE-OS 34 28 524 sind heteroaromatische Alkyldiphosphonate beschrieben. Es wurde nun gefunden, daß analoge Derivate dieser Verbindungen, in denen der Heterocyclus vollstaendig oder teilhydriert ist, auch diese Wirkung zeigen und darueber hinaus als gute Calciumkomplexbildner zur breiteren Behandlung von Calciumstoffwechselstoerungen geeignet sind. Sie lassen sich vor allem sehr gut dort einsetzen, wo der Knochenauf- und -abbau gestoert ist, d.h. sie sind geeignet zur Behandlung von Erkrankungen des Skelettsystems wie z.B. Osteoporose, Morbus Paget, Morbus Bechterew u.a.

Aufgrund dieser Eigenschaften finden sie aber auch Verwendung in der Therapie von Knochenmetastasen, der Urolithiasis und zur Verhinderung heterotoper Ossifikationen. Durch ihre Beeinflussung des Calciumstoffwechsels bilden sie weiterhin eine Grundlage fuer die Behandlung der rheumatoiden Arthritis, der Osteoarthritis und der degenerativen Arthrose. Gegenstand der vorliegenden Erfindung sind demnach Diphosphonate der allgemeinen Formel I

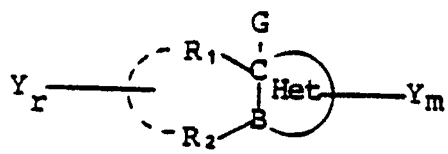

(I),

in der

Het einen Pyrrolidin- oder Piperidin-Ring,
B ein Stickstoffatom,
$R_1$ Wasserstoff,
$R_2$ Wasserstoff oder eine $C_1$-$C_4$-Alkylgruppe,
Y den Rest

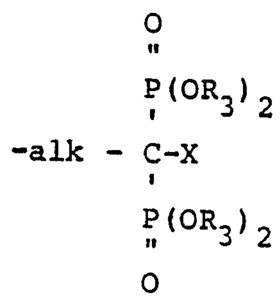

wobei $R_3$ Wasserstoff oder $C_1$-$C_4$-Alkyl,
alk eine geradkettige oder verzweigte Alkylenkette mit 1 - 6 Kohlenstoffatimen, die nicht von dem Stickstoffatom ausgehen darf,
X Hydroxy,
G Wasserstoff,
r = 0 und m = 1

bedeuten,

sowie deren pharmakologisch unbedenkliche Salze.

Niederes Alkyl bedeutet einen Kohlenwasserstoffrest mit 1 - 4 Kohlenstoffatomen, bevorzugt die Methyl-, Ethyl- und Isopropylgruppe.

Die Alkylenkette in alk ist bevorzugt Methylen, Ethylen oder Propylen.

Die Verbindungen koennen als Stereoisomerengemische oder als reine cis- bzw. trans-Isomere vorliegen.

Asymmetrische Kohlenstoffatome koennen die R-, S- oder R,S- Konfiguration besitzen.

Verbindungen der allgemeinen Formel I werden nach an sich bekannten Verfahren hergestellt.

Fuer den Fall, daß X (in Y) in der allgemeinen Formel I OH bedeutet, stellt man die Substanzen vorzugsweise dadurch her, daß man

a) eine Carbonsaeure der allgemeinen Formel II

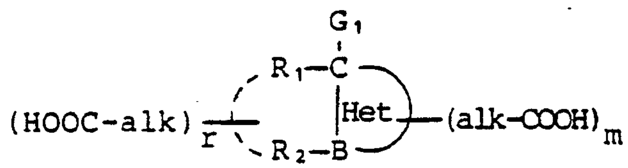 (II),

in der Het, B, $R_1$, $R_2$, alk, m und r die oben angegebenen Bedeutungen haben, $G_1$ Wasserstoff oder die Gruppierung $(alk\text{-}COOH)_s$ bedeutet, wobei alk und s die oben angegebenen Bedeutungen haben, mit einem Gemisch aus phosphoriger Saeure oder Phosphorsaeure und einem Phosphorhalogenid umsetzt und anschließend zur freien Diphosphonsaeure verseift, oder

b) ein Carbonsaeurechlorid der allgemeinen Formel III

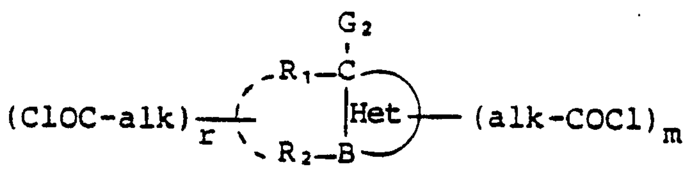 (III),

in der Het, B, $R_1$, $R_2$, alk, m und r die oben angegebenen Bedeutungen haben, $G_2$ Wasserstoff oder die Gruppierung $(alk\text{-}COCl)_s$ bedeutet, wobei alk und s die oben angegebenen Bedeutungen haben, mit einem Trialkylphosphit der allgemeinen Formel IV

$$P(OR')_3 \qquad (IV),$$

in der R′ niederes Alkyl bedeutet, zu einem Acylphosphonat der allgemeinen Formel V

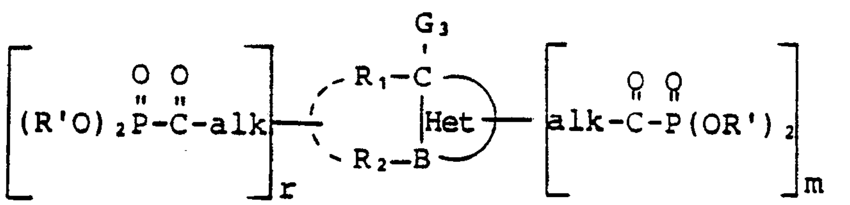 (V),

in der Het, B, $R_1$, $R_2$, R′, alk, m und r die oben angegebenen Bedeutungen haben, $G_3$ Wasserstoff oder die Gruppierung $[alk\text{-}CO\text{-}P(O)(OR')_2]_s$ bedeutet, wobei alk, R′ und s die oben angegebenen Bedeutungen haben,

umsetzt, anschließend mit einem Dialkylphosphit der allgemeinen Formel VI

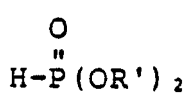 (VI),

in der R′ die oben angegebene Bedeutung hat, zu einem Diphosphonat der allgemeinen Formel VII

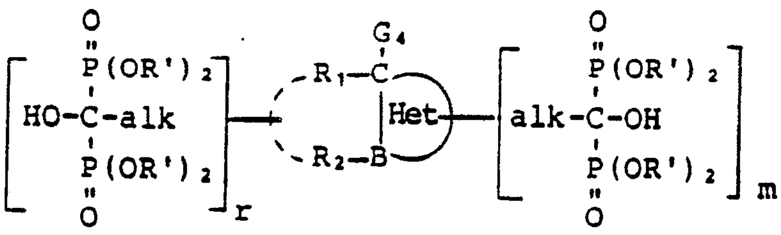

(VII),

in der Het, B, $R_1$, $R_2$, R', alk, m und r die oben angegebenen Bedeutungen haben, wobei $G_4$ Wasserstoff oder die Gruppierung [alk-C(OH)[P(O)(OR')$_2$]$_2$]$_s$ bedeutet, wobei alk, R', und s die oben angegebenen Bedeutungen haben, reagieren laeßt und gegebenenfalls die entstandenen Tetraester zu Diestern oder Saeuren der allgemeinen Formel I verseift,

oder

**Verbindungen der allgemeinen Formel XIII**

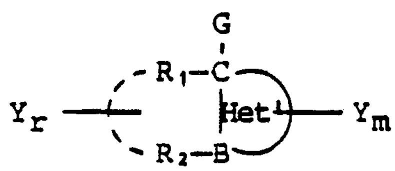

(XIII),

in der B, $R_1$, $R_2$, G, Y, m und r die oben angegebenen Bedeutungen haben und Het' einen heteroaromatischen Fuenf- oder Sechsring mit einem oder zwei Stickstoffatomen darstellt, durch Hydrieren in Verbindungen der allgemeinen Formel I ueberfuehrt,

und gegebenenfalls die entstandenen Tetraester zu Diestern oder Saeuren der allgemeinen Formel I verseift oder gewünschtenfalls die erhaltenen Verbindungen der Formel I in pharmakologisch unbedenkliche Salze überführt.

Die bei Verfahren a) eingesetzten Carbonsaeuren der allgemeinen Formel II werden mit 1 - 2, vorzugsweise 1.5 mol phosphoriger Saeure oder Phosphorsaeure und 1 - 2, vorzugsweise 1.5 mol Phosphortrihalogenid bei Temperaturen von 80 - 130 °C, vorzugsweise 100 - 110 °C umgesetzt. Man kann die Reaktion auch in Gegenwart von Verduennungsmitteln, wie Halogenkohlenwasserstoffen, insbesondere Chlorbenzol, Tetrachlorethan oder auch Dioxan durchfuehren. Die anschließende Hydrolyse erfolgt durch Kochen mit Wasser, zweckmaeßigerweise jedoch mit halbkonzentrierter Salz- oder Bromwasserstoffsaeure.

Bei Verfahren b) laeßt man das Saeurechlorid der allgemeinen Formel III mit dem Trialkylphosphit der allgemeinen Formel IV bei Temperaturen zwischen 0 und 60 °C, vorzugsweise bei 20 -40 °C zur Reaktion kommen. Man kann ohne Loesungsmittel oder auch in Gegenwart von inerten Loesungsmitteln wie Diethylether, Tetrahydrofuran, Dioxan oder auch halogenierten Kohlenwasserstoffen, wie z.B. Methylenchlorid arbeiten. Das als Zwischenprodukt entstehende Acylphosphonat der allgemeinen Formel V kann isoliert oder direkt weiter umgesetzt werden.

Die anschließende Reaktion fuehrt man in Gegenwart einer schwachen Base, vorzugsweise einem sec. Amin wie z. B. Dibutylamin bei einer Temperatur von 0 bis 60 °C, vorzugsweise bei 10 - 30 °C durch.

Bei Verfahren c) hydriert man in Gegenwart von Edelmetallkatalysatoren, z.B. Palladium oder Platin, vorzugsweise in saurem Medium z.B. in waessriger Loesung mit oder ohne Zusatz einer anorganischen oder organischen Saeure bei 1 -100 bar, vorzugsweise 1 - 10 bar bei 20 °C - 80 °C, vorzugsweise bei 20 °C - 50 °C.

Die bei den Verfahren b) und c), gegebenenfalls anfallenden Tetraalkylester koennen zu Diestern oder den freien Tetrasaeuren verseift werden. Die Verseifung zu Diestern geschieht in der Regel dadurch, daß man den Tetraalkylester mit einem Alkalihalogenid, vorzugsweise Natriumjodid in einem geeigneten Loesungsmittel wie z.B. Aceton bei Zimmertemperatur behandelt.

Hierbei entsteht das symmetrische Diester / Dinatriumsalz, das gegebenenfalls durch einen sauren Ionenaustauscher in die Diester/Disaeure umgewandelt werden kann. Die Verseifung zu freien Diphosphonsaeuren geschieht in der Regel durch Kochen mit Salz- oder Bromwasserstoffsaeure. Man kann jedoch auch eine Spaltung mit Trimethylsilylhalogenid, vorzugsweise dem Bromid oder Jodid vornehmen. Die freien Diphosphonsaeuren koennen umgekehrt durch Kochen mit Orthoameisensaeurealkylestern wieder in die Tetraalkylester ueberfuehrt werden. Die freien Diphosphonsaeuren der allgemeinen Formel I koennen als freie Saeuren oder in Form ihrer Mono-oder Dialkali- bzw. Ammoniumsalze isoliert werden. Die

Alkalisalze lassen sich in der Regel durch Umfaellen aus Wasser /Methanol oder Wasser / Aceton gut reinigen.

Die Verbindungen der allgemeinen Formel I koennen gegebenenfalls nachtraeglich ineinander ueberfuehrt werden. Sie koennen z. B. alkyliert werden oder durch hydrogenolytische Abspaltung einer N-Benzylgruppe lassen sich z.B. die entsprechenden unsubstituierten Verbindungen der allgemeinen Formel I darstellen.

Als pharmakologisch vertraegliche Salze werden vor allem Alkali- oder Ammoniumsalze verwendet, die man in ueblicher Weise z. B. durch Neutralisation der Verbindungen mit anorganischen oder organischen Basen wie z. B. Natrium- oder Kaliumhydrogencarbonat, Natronlauge, Kalilauge, waessrigem Ammoniak oder Aminen wie z. B. Trimethyl- oder Triethylamin hergestellt.

Die erfindungsgemaeßen neuen Substanzen der Formel I und ihre Salze koennen in fluessiger oder fester Form enteral und parenteral appliziert werden. Hierbei kommen alle ueblichen Applikationsformer in Frage, beispielsweise Tabletten, Kapseln, Dragees, Sirupe, Loesungen, Suspensionen etc.. Als Injektionsmedium kommt vorzugsweise Wasser zur Anwendung, welches die bei Injektionsloesungen ueblichen Zusaetze wie Stabilisierungsmittel, Loesungsvermittler und Puffer enthaelt.

Derartige Zusaetze sind z. B. Tartrat- und Citrat-Puffer, Ethanol, Komplexbildner (wie Ethylendiamintetraessigsaeure und deren nichttoxische Salze), hochmolekulare Polymere (wie fluessiges Polyethylenoxid) zur Viskositaetsregelung. Fluessige Traegerstoffe fuer Injektionsloesungen muessen steril sein und werden vorzugsweise in Ampullen abgefuellt. Feste Traegerstoffe sind z.B. Staerke, Lactose, Mannit, Methylcellulose, Talkum, hochdisperse Kieselsaeuren, hoehermolekulare Fettsaeuren (wie Staearinsaeure), Gelatine, Agar-Agar, Calciumphosphat, Magnesiumstearat, tierische und pflanzlische Fette, feste hochmolekulare Polymere (wie Polyethylenglykole); fuer orale Applikation geeignete Zubereitungen koennen gewuenschtenfalls Geschmacks- und Sueßstoffe enthalten.

Die Dosierung kann von verschiedenen Faktoren, wie Applikationsweise, Spezies, Alter und / oder individuellem Zustand abhaengen. Die taeglich zu verabreichenden Dosen liegen bei etwa 1 - 1000 mg/Mensch, vorzugsweise bei 10 -200 mg/Mensch und koennen auf einmal oder mehrere Male verteilt eingenommen werden.

Bevorzugt im Sinne der vorliegenden Erfindung sind außer den in den Beispielen genannten Verbindungen und durch Kombination aller in den Anspruechen genannten Bedeutungen der Substuenten ableitbaren Verbindungen die folgenden Diphosphonsaeuren, sowie deren Natriumsalze, Methyl und Ethylester.

1-Hydroxy-2-(piperidin-2-yl)propan-1.1-diphosphonsaeure
1-Hydroxy-2-(pyrrolidin-2-yl)ethan-1,1-diphosphonsaeure
1-Hydroxy-2-(1-methylpyrrolidin-2-yl)ethan-1,1-diphosphonsaeure
1-Hydroxy-2-(pyrrolidin-3-yl)ethan-1,1-diphosphonsaeure
1-Hydroxy-2-(1-propyl-pyrrolidin-3-yl)ethan-1,1-diphosphonsaeure
1-Hydroxy-3-(piperidin-2-yl)butan-1,1-diphosphonsaeure
1-Hydroxy-5-(piperidin-2-yl)pentan-1,1-diphosphonsaeure
1-Hydroxy-2-(piperidin-3-yl)ethan-1,1-diphosphonsaeure
1-Hydroxy-3-(piperidin-3-yl)butan-1,1-diphosphonsaeure

Die nachfolgenden Beispiele zeigen eine der Verfahrensvarianten, die zur Synthese der erfindungsgemaeßen Verbindungen verwendet werden koennen. Sie sollen jedoch nicht eine Einschraenkung des Erfindungsgegenstandes darstellen. Die Verbindungen fallen in der Regel als hochschmelzende Festprodukte an (Mono- oder Dinatriumsalz), deren Struktur durch H-, P- und gegebenenfalls durch $^{13}C$ NMR-Spektroskopie gesichert wurde. Die Reinheit der Substanzen wurde mittels C,H,N,P,S, Na-Analyse sowie durch Duennschichtelektrophorese (Cellulose, Oxalat-Puffer von pH = 4.0) bestimmt. Zur Charakterisierung der einzelnen Verbindungen werden die $M_{rel}$-Werte (= relative Mobilitaet) bezogen auf Pyrophosphat ($M_{rel}$ = 1) angegeben.

## Beispiel 1

### 1-(Dekahydro-chinolin-3-yl)methan-1-hydroxy-1,1-diphosphonsaeure

5.6 g Dekahydro-chinolin-3-carbonsaeure (hergestellt durch Verseifen des entsprechenden Ethylesters, der seinerseits durch Hydrierung des 4-Chlor-chinolin-3-carbonsaeureethylester mit Palladium auf Kohle in Eisessig bei 5 bar und 60° C dargestellt wurde) werden in 40 ml Chlorbenzol suspendiert und mit 2.9 g phosphoriger Saeure versetzt. Man erhitzt 10 Minuten auf 120° C, tropft dann 3.8 ml Phosphortrichlorid zu

und laeßt 8 Stunden bei 120 °C ruehren. Nach dem Abkuehlen filtriert man eine gelbe, amorphe Masse ab, waescht mit Ether und erhitzt den Rueckstand 5 Stunden mit 60 ml halbkonzentrierter Salzsaeure unter Rueckfluß. Nach dem Abkuehlen saugt man ab und engt das Filtrat ein. Der Rueckstand wird getrocknet und mit Aceton verruehrt. Nach 1 Stunde wird das Aceton dekantiert, der Rueckstand in 16 ml Wasser geloest und filtriert. Das Filtrat wird mit 2 N Natronlauge auf einen pH ≈ 5 gebracht und mit 150 ml Methanol versetzt. Das ausgefallene Produkt wird abgesaugt und getrocknet. Man erhaelt so 3.1 g = 26 % d. Theorie des Dinatriumsalzes x 1 mol Wasser, Fp: 270-280 °C, $M_{rel}$: 0.40.

In analoger Weise erhaelt man durch Verwendung von

a) 1-Ethoxycarbonyl-piperidin-2-ylessigsaure (Fp: 58-60 °C) die 1-Hydroxy-2-(piperidin-2-yl)ethan-1,1-diphosphonsaeure als Mononatriumsalz x 1 Wasser in einer Ausbeute von 23 %, Fp: 250-255 °C, $M_{rel}$: 0.40

b) 1-Methyl-piperidin-2-ylessigsaeure-hydrochlorid (Fp: 178-179 °C) die 1-Hydroxy-2-(1-methyl-piperidin-2-yl)ethan-1,1-diphosphonsaeure als Mononatriumsalz x 1 Wasser in einer Ausbeute von 28 %, Fp: 155-160 °C, $M_{rel}$: 0.40

c) 3-(Pyrrolidin-2-yl)propionsaeure (Can.J.Chem. 57 , 1977) die 1-Hydroxy-3-(pyrrolidin-2-yl)propan-1,1-diphosphonsaeure als Mononatriumsalz x 1 Wasser in einer Ausbeute von 39 %, Fp: 275-280 °C, $M_{rel}$: 0.50

## Beispiel 2

### 1-Hydroxy-3-(piperidin-2-yl)propan-1,1-diphosphonsaeure

1.5 g 3-(Piperidin-2-yl)propionsaeure-hydrochlorid (Fp: 193-195 °C, durch Hydrierung der 3-(2-Pyridyl)-propionsaeure in salzsaurem Medium in Gegenwart von Plationoxid hergestellt) und 1.3 g phosphorige Saeure werden bei 80 °C zusammengeschmolzen. Man laeßt abkuehlen, tropft 1.4 ml Phosphortrichlorid zu und erhitzt weitere 20 Stunden auf 90 °C. Nach dem Abkuehlen gibt man vorsichtig 20 ml Wasser zu und laeßt 7 Stunden unter Rueckfluß erhitzen. Die abgekuehlte Loesung wird mit Kohle behandelt, filtriert und das Filtrat eingeengt. Nach dem Trocknen wird der Rueckstand mehrmals mit Aceton verruehrt, dann in Wasser aufgenommen, die Loesung mit 2 N Natronlauge auf einen pH≈ 5 gebracht und mit Methanol versetzt. Man ruehrt einige Zeit unter Eiskuehlung, saugt den Niederschlag ab und trocknet ihn. Man erhaelt so 1.05 g = 37 % d. Theorie des Mononatriumsalzes x 2 Wasser, Fp: 270-274 °C, $M_{rel}$: 0.41

## Beispiel 3

### 1-Hydroxy-1-(4-piperidinyl)methan-1,1-diphosphonsaeure

12.9 g Piperidin-4-carbonsaeure und 12.3 g phosphorige Saeure werden in 130 ml Chlorbenzol suspendiert, auf 100 °C gebracht und tropfenweise mit 26.1 ml Phosphortrichlorid versetzt. Die Reaktion wird dann bei 130 °C weitergefuehrt. Nach 25 Stunden destilliert man das Chlorbenzol ab, versetzt mit 170 ml 6 N Salzsaeure und kocht 13 Stunden. Man filtriert, konzentriert das Filtrat und gießt in 2 1 Aceton. Der schmierige Niederschlag wird mit Isopropanol kristallin (7.7 g = 28 %). Nach Umkristallisieren aus Wasser erhaelt man 3.6 g = 13 % als Monohydrat vom Fp. 238 °C Sintern/246-248 °C. $M_{rel}$: 0.41

### 3 a)

In analoger Weise erhaelt man aus der 3-(4-piperidinyl)-propionsaeure (Biochem.J. 46 , 192) die 1-Hydroxy-3-(4-piperidinyl)propan-1,1-diphosphonsaeure mit 35 % Ausbeute, als Hydrochlorid, Fp. 230 °C Sintern/234-238 °C Blasenbildung (aus waessr. Methanol). $M_{rel}$: 0.40.

## Ansprüche

1. Diphosphonsäure-Derivate der Formel I

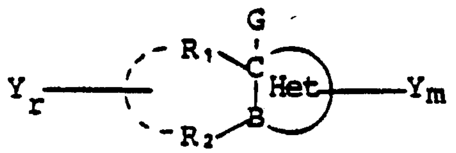

(I),

in der
Het einen Pyrrolidin- oder Piperidin-Ring,
B ein Stickstoffatom,
$R_1$ Wasserstoff,
$R_2$ Wasserstoff oder eine $C_1$-$C_4$-Alkylgruppe,
Y den Rest

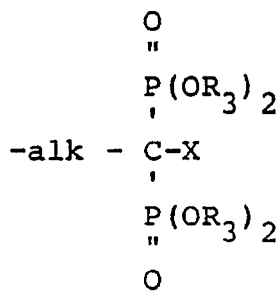

wobei $R_3$ Wasserstoff oder $C_1$-$C_4$-Alkyl,
alk eine geradkettige oder verzweigte Alkylenkette mit 1 - 6 Kohlenstoffatimen, die nicht von dem Stickstoffatom ausgehen darf,
X Hydroxy,
G Wasserstoff,
r = 0 und m = 1 bedeuten,
sowie deren pharmakologisch unbedenkliche Salze.

2. Verbindungen der Formel I gemäß Anspruch 1, in der
$R_1$ Wasserstoff,
$R_2$ Wasserstoff, Methyl oder Propyl,
alk eine $C_1$-$C_2$-Alkylenkette und
$R_3$, Het, B, Y, X, G, r und m die angegebenen Bedeutungen haben.

3. Die Verbindungen gemäß Anspruch 1 oder 2
1-Hydroxy-2-(piperidin-2-yl)-ethan-1,1-diphosphonsäure,
1-Hydroxy-2-(1-methyl-piperidin-2-yl)-ethan-1,1-diphosphonsäure und
1-Hydroxy-3-(pyrrolidin-2-yl)-propan-1,1-diphosphonsäure sowie deren pharmakologisch verträgliche Salze.

4. Verfahren zur Herstellung von Diphosphonsäure-Derivaten gemäß Anspruch 1, 2 oder 3 sowie deren pharmakologisch verträgliche unbedenkliche Salze , dadurch gekennzeichnet, daß
a) eine Carbonsaeure der allgemeinen Formel II

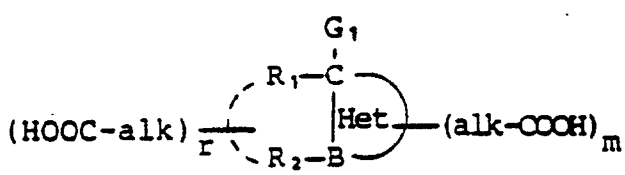

(II),

in der Het, B, $R_1$, $R_2$, alk, m und r die oben angegebenen Bedeutungen haben, $G_1$ Wasserstoff oder die Gruppierung $(alk\text{-}COOH)_S$ bedeutet, wobei alk und s die oben angegebenen Bedeutungen haben, mit einem Gemisch aus phosphoriger Saeure oder Phosphorsaeure und einem Phosphorhalogenid umsetzt und anschließend zur freien Diphosphonsaeure verseift, oder

b) ein Carbonsaeurechlorid der allgemeinen Formel III

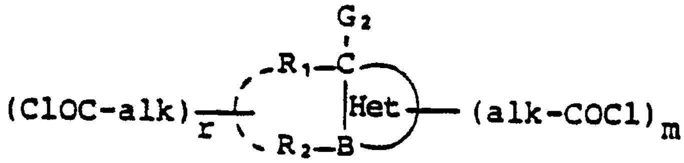

(III),

in der Het, B, $R_1$, $R_2$, alk, m und r die oben angegebenen Bedeutungen haben, $G_2$ Wasserstoff oder die Gruppierung $(alk\text{-}COCl)_S$ bedeutet, wobei alk und s die oben angegebenen Bedeutungen haben, mit einem Trialkylphosphit der allgemeine Formel IV

$$P(OR')_3 \qquad (IV),$$

in der R' niederes Alkyl bedeutet, zu einem Acylphosphonat der allgemeinen Formel V

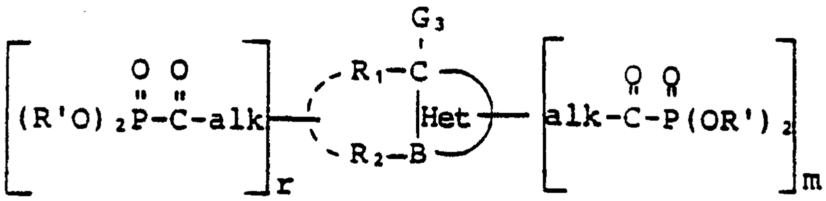

(V),

in der Het, B, $R_1$, $R_2$, R', alk, m und r die oben angegebenen Bedeutungen haben, $G_3$ Wasserstoff oder die Gruppierung $[alk\text{-}CO\text{-}P(O)(OR')_2]_S$ bedeutet, wobei alk, R' und s die oben angegebenen Bedeutungen haben,

umsetzt, anschließend mit einem Dialkylphosphit der allgemeinen Formel VI

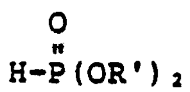

(VI),

in der R' die oben angegebene Bedeutung hat, zu einem Diphosphonat der allgemeinen Formel VII

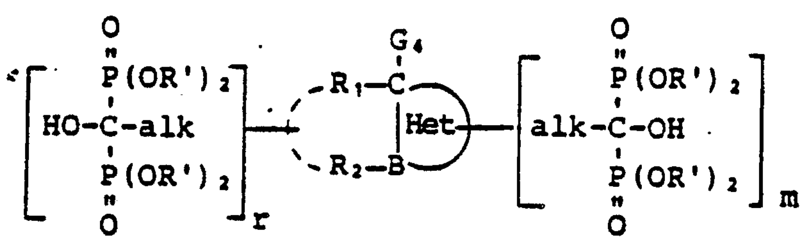

(VII),

in der Het, B, $R_1$, $R_2$, R', alk, m und r die oben angegebenen Bedeutungen haben, wobei $G_4$ Wasserstoff oder die Gruppierung $[alk\text{-}C(OH)[P(O)(OR')_2]_2]_S$ bedeutet, wobei alk, R', und s die oben angegebenen Bedeutungen haben, reagieren laeßt und gegebenenfalls die entstandenen Tetraester zu Diestern oder Saeuren der allgemeinen Formel I verseift,

oder

Verbindungen der allgemeinen Formel XIII

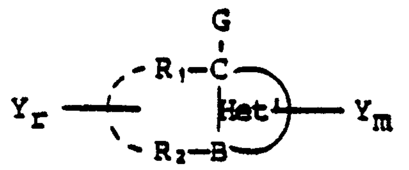 (XIII),

in der B, $R_1$, $R_2$, G, Y, m und r die oben angegebenen Bedeutungen haben und Het' einen heteroaromatischen Fuenf- oder Sechsring mit einem oder zwei Stickstoff-atomen darstellt, durch Hydrieren in Verbindungen der allgemeinen Formel I ueberfuehrt, und gegebenenfalls die entstehenden Tetraester zu Diestern oder Säuren der Formel I überführt oder die erhaltenen Verbindungen der Formel I in andere Verbindungen der Formel I umwandelt oder gewünschtenfalls die Verbindungen der Formel I in ihre pharmakologisch unbedenklichen Salze überführt.

**5.** Arzneimittel enthaltend eine Verbindung gemaeß Anspruch 1, 2 oder 3 neben ueblichen Traeger- und Hilfsstoffen.

**6.** Verbindungen gemaeß Anspruch 1, 2 oder 3 zur Behandlung von Calciumstoffwechselstoerungen.

## Claims

**1.** Diphosphonic acid derivatives of general formula I

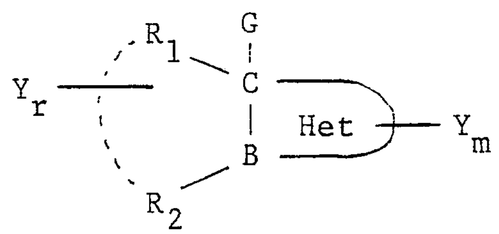 (I)

in which Het signifies a pyrrolidine or piperidine ring, B a nitrogen atom, $R_1$ hydrogen, $R_2$ hydrogen or a $C_1$-$C_4$-alkyl group, Y the radical

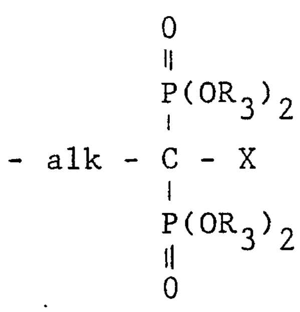

whereby $R_3$ signifies hydrogen or $C_1$-$C_4$-alkyl, alk a straight-chained or branched alkylene chain with 1 - 6 carbon atoms which must not start from the nitrogen atom, X hydroxyl, G hydrogen, r = 0 and m = 1, as well as their pharmacologically acceptable salts.

**2.** Compounds of the formula I according to claim 1, in which $R_1$ is hydrogen, $R_2$ hydrogen, methyl or propyl, alk a $C_1$-$C_2$-alkylene chain and $R_3$, Net, B, Y, X, G, r and m have the given meanings.

**3.** The compounds according to claim 1 or 2
1-hydroxy-2-(piperidin-2-yl)-ethane-1,1-diphosphonic acid
1-hydroxy-2-(1-methylpiperidin-2-yl)-ethane-1,1-diphosphonic acid
1-hydroxy-3-(pyrrolidin-2-yl)-propane-1,1-diphosphonic acid as well as their pharmacologically acceptable salts.

**4.** Process for the preparation of diphosphonic acid derivatives according to claim 1, 2 or 3, as well as of their pharmacologically acceptable salts, characterised in that

a) a carboxylic acid of the general formula II

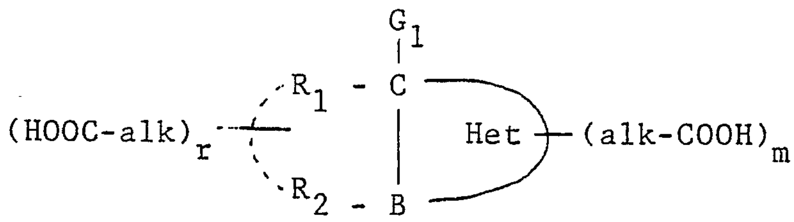

(II)

in which Het, B, $R_1$, $R_2$, alk, m and r have the above-given meanings, $G_1$ signifies hydrogen or the grouping $(alk\text{-}COOH)_S$, whereby alk and s have the above-given meanings, is reacted with a mixture of phosphorous acid or phosphoric acid and a phosphorus halide and subsequently saponified to the free diphosphonic acid, or

b) a carboxylic acid chloride of the general formula III

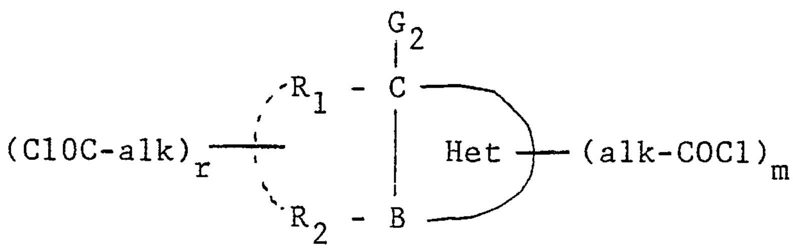

(III)

in which Het, B, $R_1$, $R_2$, alk, m and r have the above-given meanings, $G_2$ signifies hydrogen or the grouping $(alk\text{-}COCl)_S$, whereby alk and s have the above-given meanings, is reacted with a trialkyl phosphite of the general formula IV

$$P(OR')_3 \qquad (IV),$$

in which R' signifies lower alkyl, to give an acyl phosphonate of the general formula V

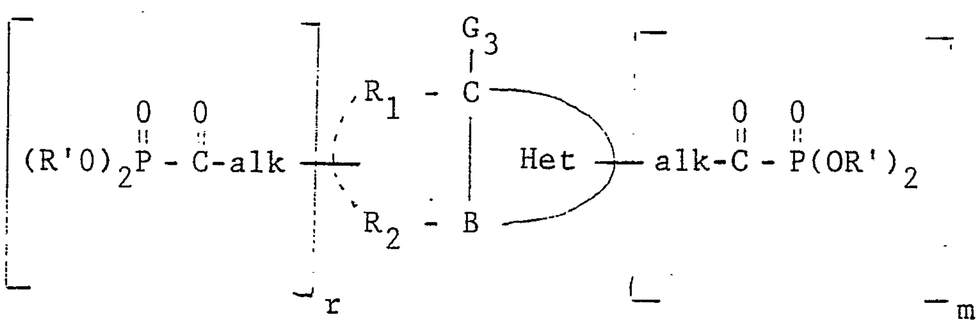

(V),

in which Het, B, $R_1$, $R_2$, R', alk, m and r have the above-given meanings, $G_3$ signifies hydrogen or the grouping $[alk\text{-}CO\text{-}P(O)(OR')_2]_S$, whereby alk, R' and s have the above-given meanings, subsequently allows to react with a dialkyl phosphite of the general formula VI

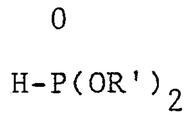 (VI),

in which R' has the above-given meaning, to give a diphosphonate of the general formula VII

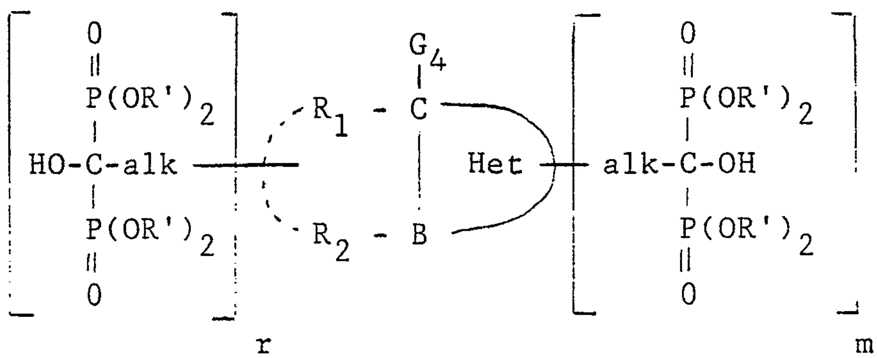 (VII),

in which Het, B, $R_1$, $R_2$, R', alk, m and r have the above-given meanings, whereby $G_4$ signifies hydrogen or the grouping $[alk\text{-}C(ON)[P(O)(OR')_2]_2]_S$, whereby alk, R' and s have the above-given meanings, and the resultant tetraester optionally saponified to diesters or acids of general formula I, or

c) compounds of the general formula XIII

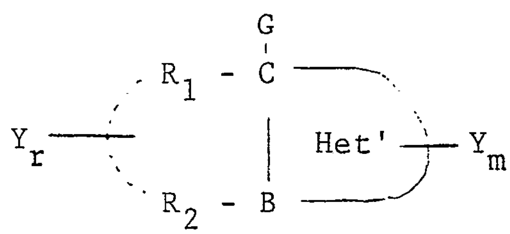 (XIII)

in which B, $R_1$, $R_2$, G, Y, m and r have the above-given meanings and Het' represents a heteroaromatic five- or six-membered ring with one or two nitrogen atoms, are converted by hydrogenation into compounds of general formula I, and the resultant tetraesters optionally converted into diesters or acids of the formula I or the compounds obtained of general formula I converted into other compounds of the formula I or, if desired, the compound of the formula I converted into their pharmacologically acceptable salts.

**5.** Medicaments containing a compound according to claim 1, 2 or 3, besides usual carrier and adjuvant materials.

**6.** Compounds according to claim 1, 2 or 3 for the treatment of calcium metabolism disturbances.

## Revendications

**1.** Dérivés d'acides diphosphoniques de formule I

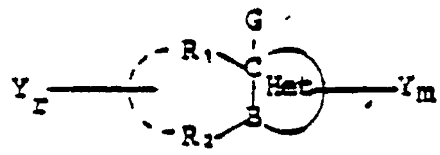

(I),

où

Het représente un cycle pyrrolidine ou pipéridine,
B représente un atome d'azote,
$R_1$ représente un atome d'hydrogène,
$R_2$ représente un atome d'hydrogène ou un groupe alkyle en $C_1$-$C_4$,
Y représente le reste

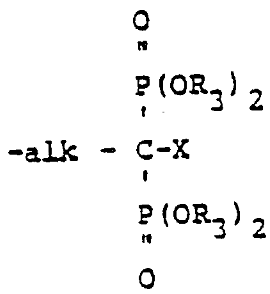

où

$R_3$ représente un atome d'hydrogene ou un groupe alkyle en $C_1$-$C_4$,
alk représente une chaîne alkylène linéaire ou ramifiée, comportant 1-6 atomes de carbone, qui ne doit pas partir de l'atome d'azote,
X représente un groupe hydroxy,
G représente un atome d'hydrogène,
r = 0 et m = 1, ainsi que leurs sels pharmacologiquement acceptables.

**2.** Composés de formule I selon la revendication 1, dans lesquels
$R_1$ représente un atome d'hydrogène,
$R_2$ représente un atome d'hydrogène, un groupe méthyle ou propyle,
alk représente une chaîne alkylène en $C_1$-$C_2$, et
$R_3$, Het, B, Y, X, G, r et m ont les significations mentionnées ci-dessus.

**3.** Composés selon la revendication 1 ou 2, qui sont
l'acide 1-hydroxy-2(pipéridin-2-yl)-éthane-1,1-diphosphonique,
l'acide 1-hydroxy-2-(1-méthyl-pipéridin-2-yl)-éthane-1,1-diphosphonique, et
1'acide 1-hydroxy-3-(pyrrolidin-2-yl)-propane-1,1-diphosphonique, ainsi que leurs sels pharmacologiquement acceptables.

**4.** Procédé de préparation des dérivés d'acides diphosphoniques selon la revendication 1, 2 ou 3, ainsi que de leurs sels pharmacologiquement acceptables, caractérisé en ce que
a) on fait réagir un acide carboxylique de formule générale II

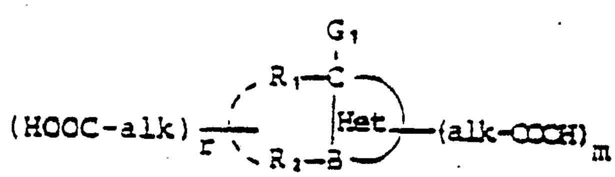

(II),

où Het, B, $R_1$, $R_2$, alk, m et r ont les significations mentionnées ci-dessus, $G_1$ représente un atome d'hydrogène ou le groupement $(alk\text{-}COOH)_S$, dans lequel alk et s ont les significations mentionnées

ci-dessus, avec un mélange d'acide phosphoreux ou d'acide phosphorique et d'un halogénure de phosphore, et ensuite, on saponifie en acide diphosphonique libre, ou

b) on fait réagir un chlorure d'acide carboxylique de formule générale III

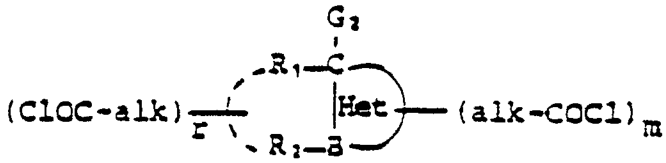 (III),

où Het, B, $R_1$, $R_2$, alk, m et r ont les significations mentionnées ci-dessus, $G_2$ représente un atome d'hydrogène ou le groupement $(alk\text{-}COCl)_S$, dans lequel alk et s ont les significations mentionnées ci-dessus, avec un trialkylphosphite de formule générale IV

$$P(OR')_3 \qquad (IV),$$

où R' représente un groupe alkyle inférieur, pour obtenir un phosphonate d'acyle de formule générale V

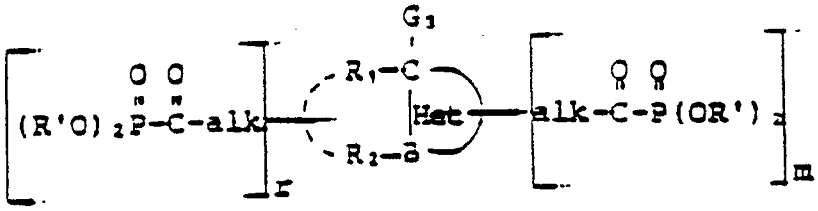 (V),

où Het, B, $R_1$, $R_2$, R', alk, m et r ont les significations mentionnées ci-dessus, $G_3$ représente un atome d'hydrogène ou le groupement $[alk\text{-}CO\text{-}P(O)(OR')_2]_S$, dans lequel alk, R' et s ont les significations mentionnées ci-dessus,

ensuite on fait réagir le composé obtenu avec un dialkylphosphite de formule générale VI

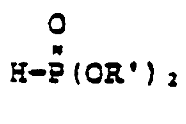 (VI),

où R' a la signification mentionnée ci-dessus, pour obtenir un diphosphonate de formule générale VII

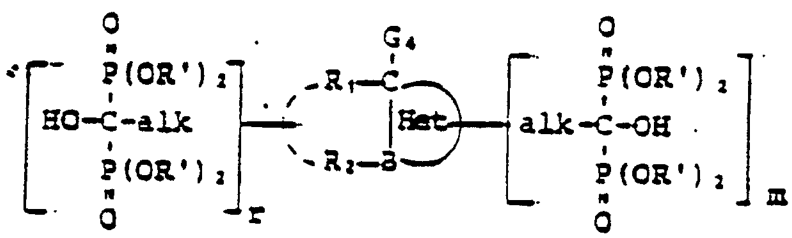 (VII),

où Het, B, $R_1$, $R_2$, R', alk, m et r ont les significations mentionnées ci-dessus, $G_4$ représente un atome d'hydrogène ou le groupement $[alk\text{-}C(OH)[P(O)(OR')_2]_2]_S$, dans lequel alk, R' et s ont les significations mentionnées ci-dessus, et éventuellement, on saponifie le tétraester formé en diester ou en acide de formule générale I,

ou

on transforme des composés de formule générale XIII

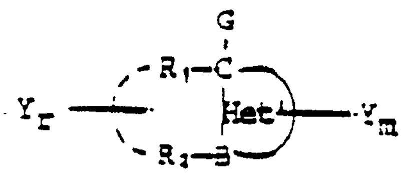

(XIII),

où

B, $R_1$, $R_2$, G, Y, m et r ont les significations mentionnées ci-dessus et Het représente un cycle hétéroaromatique à cinq ou six chaînons, comportant un ou deux atomes d'azote, par hydrogénation, en composés de formule générale I,

et éventuellement, on transforme le tétraester obtenu en diester ou en acide de formule I et transforme les composés de formule I obtenus en d'autres composés de formule I, ou éventuellement, on transforme les composés de formule I en leurs sels pharmacologiquement acceptables.

**5.** Médicament contenant un composé selon la revendication 1, 2 ou 3, en plus des supports et adjuvants usuels.

**6.** Composé selon la revendication 1, 2 ou 3, pour le traitement des troubles du métabolisme calcique.